# EUROPEAN PATENT APPLICATION

(11) **EP 3 213 671 A1**
(43) Date of publication of application: **06.09.2017**
(21) Application number: 14904798.7
(22) Date of filing: 31.10.2014
(51) Int. Cl.: A61B 5/00, A61B 5/11

(54) **STATE DISPLAY METHOD, PROGRAM, AND STATE DISPLAY DEVICE**

(71) Applicant: Fujitsu Limited, Kawasaki-shi, Kanagawa 211-8588 (JP)
(72) Inventor: YAMAJI, Takayuki, Kawasaki-shi Kanagawa 211-8588 (JP); MASUDA, Yuta, Kawasaki-shi Kanagawa 211-8588 (JP)
(74) Representative: Hoffmann Eitle
(86) International application number: PCT/JP2014/079057
(87) International publication number: WO 2016/067450

(57) **Abstract**

A disclosed state display method includes: obtaining first data representing first temporal change of a degree of a depth of sleep in a sleep state and second data representing second temporal change of a degree of an amount of activity in a non-sleep state, the first data and the second data being data measured for a subject; generating, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and displaying the generated graph.

## Description

### Technical Field

This invention relates to a technique for visualizing states of vital activities.

### Background Technology

One patent document discloses an example in which a line graph representing transition of action indicators in chronological order and a band graph representing time zones during a rest, activities, sleep and a meal are displayed on one screen. In this example, a user can grasp a degree of activity during the activity, but the user cannot grasp a degree of sleep during the sleep.

Moreover, another patent document discloses an example of displaying a graph representing a sleep state in chronological order. In this example, the user can grasp a degree of sleep during the sleep, but the user cannot grasp a degree of activity during the activity.

### Prior technical documents

### Patent Documents

Patent Document 1: Japanese Laid-open Patent Publication No. 2006-129887
Patent Document 2 : Japanese Laid-open Patent Publication No. 2008-006005

### Summary of the Invention

### Object to be solved by the Invention

As one aspect, an object of this invention is to provide a technique for enabling a user to chronologically grasp a relation between a depth of sleep and an amount of activity.

### Means for solving the Problem

A state display method related to one aspect includes: obtaining both first data that represents first temporal change of a degree of a depth of sleep in a sleep state and second data that represents second temporal change of a degree of an amount of activity in a non-sleep state, the first data and the second data being data measured for a subject; generating, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and displaying the generated graph.

A state display method related to one aspect includes: displaying a first object that represents a depth of sleep in a first direction perpendicular to a time axis in a case where measurement results of vital activity of a subject represent a sleep state, when displaying temporal transition of states of the subject based on the measurement results; and displaying a second object that represents an intensity of activity in a second direction which is perpendicular to the time axis and is opposite to the first direction in a case where the measurement results represent a non-sleep state.

### Effect of the Invention

It becomes possible to enable a user to chronologically grasp a relation between a depth of sleep and an amount of activity.

### Brief description of the drawings

FIG. 1 is a diagram depicting an example of network configuration;
FIG. 2 is a diagram depicting an example of a graph image;
FIG. 3 is a diagram depicting an example of module configuration of a display processing apparatus related to a first embodiment;
FIG. 4 is a diagram depicting an example of a main processing flow related to the first embodiment;
FIG. 5 is a diagram depicting an example of first data;
FIG. 6 is a diagram depicting an example of second data;
FIG. 7 is a diagram depicting an example of configuration of a graph image;
FIG. 8 is a diagram depicting an example of a generation processing flow;
FIG. 9 is a diagram depicting an example of a first rendering processing flow;
FIG. 10 is a diagram depicting an example of a first parts image;
FIG. 11 is a diagram for explaining a paste destination of the first parts image;
FIG. 12 is a diagram depicting an example of a second rendering processing flow;
FIG. 13 is a diagram depicting an example of a second parts image;
FIG. 14 is a diagram for explaining a paste destination of the second parts image;
FIG. 15 is a diagram depicting an example of module configuration of the display processing apparatus related to a second embodiment;
FIG. 16 is a diagram depicting an example of the main processing flow related to the second embodiment;
FIG. 17 is a diagram depicting an example of a display processing flow (A);
FIG. 18 is a diagram depicting an example of a first superimposition display processing flow;
FIG. 19 is a diagram depicting an example of a second superimposition display processing flow;
FIG. 20 is a diagram depicting an example of a display processing flow (B);
FIG. 21 is a functional block diagram of a computer; and
FIG. 22 is a diagram depicting an example of hardware configuration of a mobile terminal device.

### Mode for carrying out the invention

### [Embodiment 1]

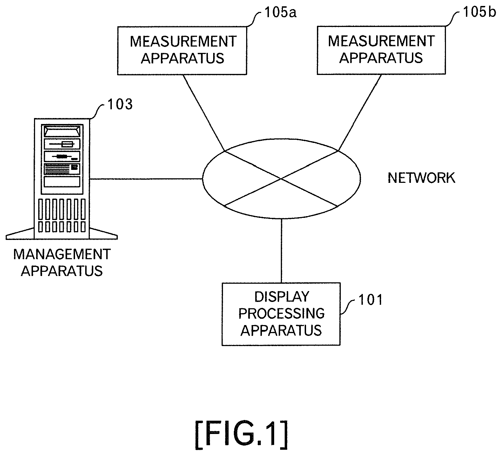

FIG. 1 illustrates an example of network configuration. A displayprocessingapparatus 101 is anapparatus that visualizes states of vital activity of a subject (also referred to as a target). The display processing apparatus 101 has a display device such as an LCD (Liquid Crystal Display), for example. Specifically, the display processing apparatus 101 graphically displays a level of a depth of sleep and a level of an amount of activity. The network is, for example, amobilecommunicationnetwork, the Internet, aLAN (Local Area Network) or the like.

A measurement apparatus 105a is an apparatus that measures the depth of sleep. The measurement apparatus 105a measures, for example, an electroencephalogram, a pulse, a body temperature, a body motion, a myoelectricity, a blood pressure, a respiratory rate, or the like of a subject. The level of the depth of sleep is determined based on measurement results in the measurement apparatus 105a. The level of the depth of sleep determined in the measurement apparatus 105a is sent to the management apparatus 103 via the network, and the management apparatus 103 manages the level of the depth of sleep for each subject. Alternatively, the measurement results maybe sent from the measurement apparatus 105a to the management apparatus 103 via the network, and the management apparatus 103 may determine the level of the depth of sleep. As an example of the level of the depth of sleep, for example, sleep stages may be used.

A measurement apparatus 105b is an apparatus that measures the amount of activity of a subject. For example, when the measurement apparatus 105b is a mobile device such as a smartphone or a wearable terminal, acceleration related to the movement of the measurement apparatus 105b itself is measured. Alternatively, for example, when the measurement apparatus 105b is an apparatus installed in a living environment or a working environment, a position, a posture, a heart rate and the like of the subj ect are measured. The measurement apparatus 105b installed in this manner may measure the position and the posture of the subject by analyzing the photographed image or reflection of the microwave irradiated by itself. The level of the amount of activity is determined based on the measurement results in the measurement apparatus 105b. The level of the amount of activity determined in the measurement apparatus 105b is sent to the management apparatus 103 via the network, and the management apparatus 103 manages the level of the amount of activity for each subject. Alternatively, the measurement results may be sent from the measurement apparatus 105 b to the management apparatus 103 via the network, and the management apparatus 103 may determine the level of the amount of activity. For example, METs (Metabolic equivalents) may be used as an example of the level of the amount of activity.

Further, the level of the amount of activity may be determined based on the measurement results in the measurement apparatus 105a. Further, the level of the depth of sleep may be determined based on the measurement results in the measurement apparatus 105b. The measurement apparatus 105a and the measurement apparatus 105b may be the same apparatus.

Then, the display processing apparatus 101 obtains, from the management apparatus 103, data for the level of the depth of sleep (hereinafter, referred to as first data) and data for the level of the amount of activity (hereinafter referred to as second data) for the specific subject via the network.

The display processing apparatus 101 may obtain the first data directly from the measurement apparatus 105a. Likewise, the display processing apparatus 101 may obtain the second data directly from the measurement apparatus 105a. Similarly, the display processing apparatus 101 may obtain the second data directly from the measurement apparatus 105b. Similarly, the display processing apparatus 101 may obtain the first data directly from the measurement apparatus 105b.

The display processing apparatus 101 may also serve as the measurement apparatus 105a. The display processing apparatus 101 may also serve as the measurement apparatus 105b. The display processing apparatus 101 may also serve as the management apparatus 103. When the display processing apparatus 101 itself performs measurement and generates the first data and the second data, it is not necessary to perform communication via the network.

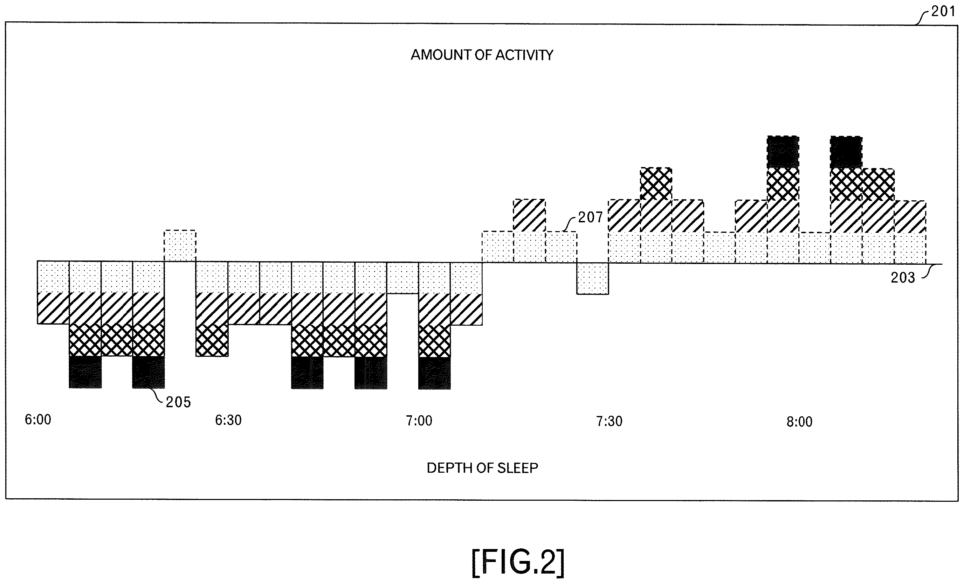

Next, a graph image displayed in the display processing apparatus 101 will be explained. FIG. 2 illustrates an example of a graph image. In the graph image 201, a time axis 203 is provided in the horizontal direction.

The bar 205 arranged downward from the time axis 2 03 represents the level of the depth of sleep. In other words, the bar graph representing the level of the depth of sleep represents the level of the depth of sleep by a distance from the time axis 203 to the end of the bar 205. A color of a hue (for example, "blue") that has an image of sleep is used for the bar 205 representing the level of the depth of sleep. Furthermore, a color of the bar 205 representing the level of the depth of sleep becomes gradually darker as going away from the time axis 203. In FIG. 2 and the subsequent figures, the bars 205 assumed to be occupied by colors with "blue" hue are surrounded by solid lines.

The bar 207 arranged upward from the time axis 203 represents the level of the amount of activity. In other words, the bar graph representing the level of the amount of activity represents the level of the amount of activity by a distance from the time axis 203 to the end of the bar 207. A color of a hue (for example, "orange") that has an image of activity is used for the bar 207 representing the level of the amount of activity. Furthermore, a color of the bar 207 representing the level of the amount of activity becomes gradually darker as going away from the time axis 2 03. In FIG. 2 and the subsequent figures, the bar 207 assumed to be occupied by colors with "orange" hue is surrounded by broken lines. By making a color of the bar 205 representing the level of the depth of sleep and a color of the bar 207 representing the level of the amount of activity different from each other, it makes it easier to recognize by sight the display is related to which of the sleep and the activity. Although a bar graph is illustrated in FIG. 2, the form of the graph is not limited to this. For example, it may be scored with a line chart. In addition, in FIG. 2, the time axis 203 is an example in which passage of time is illustrated from left to right in the figure, but it is not limited to this. For example, passage of time may be illustrated from the top to the bottom in the figure. In this case, for example, the right side of the time axis may be an area representing the level of the amount of activity, and the left side of the time axis may be an area representing the level of the depth of sleep. Of course, a case where the amount of activity or the depth of sleep is on the right side of the time axis and the other one is on the left side of the time axis is allowed.

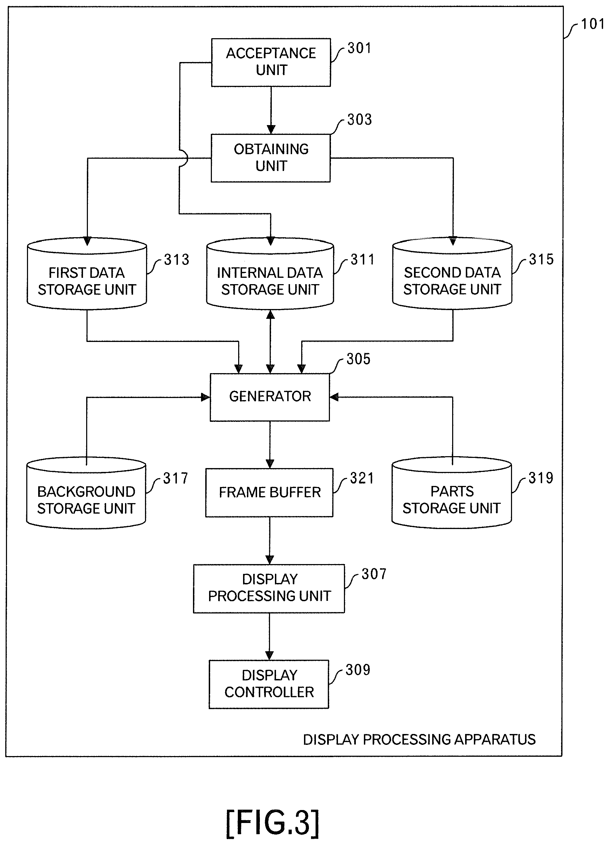

Next, module configuration of the display processing apparatus 101 will be explained. FIG. 3 illustrates an example of module configuration of the display processing apparatus 101 related to the first embodiment. The display processing apparatus 101 includes an acceptance unit 301, an obtaining unit 303, a generator 305, a display processing unit 307, a display controller 309, an internal data storage unit 311, a first data storage unit 313, a second data storage unit 315, a background storage unit 317, parts storage unit 319 and a frame buffer 321.

The acceptance unit 301 accepts an instruction from a user. The obtaining unit 303 obtains the first data and the second data. The generator 305 generates a graph image. The display processing unit 307 performs processing for displaying a graph image. A display controller 309 controls the display device.

The internal data storage unit 311 stores parameters to be used internally. The first data storage unit 313 stores the first data. The second data storage unit 315 stores the second data. The background storage unit 317 stores background image data. The parts storage unit 319 stores a parts image. The frame buffer 321 stores a graph image.

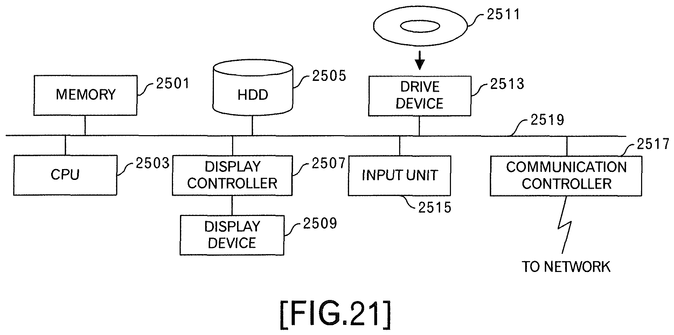

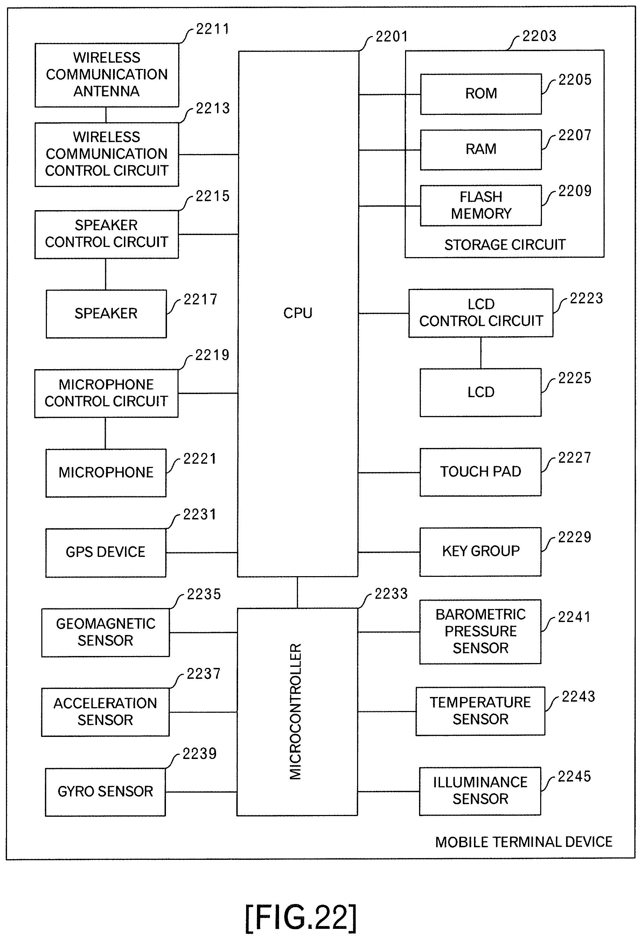

The above-described acceptance unit 301, obtaining unit 303, generator 305, display processing unit 307, and display controller 309 are realized by using hardware resources (for example, FIG. 21 or FIG. 22) and a program that causes a processor to execute processing described below.

The above-described internal data storage unit 311, first data storage unit 313, second data storage unit 315, background storage unit 317, parts storage unit 319 and frame buffer 321 are realized by using hardware resources (for example, FIG. 21 or FIG. 22).

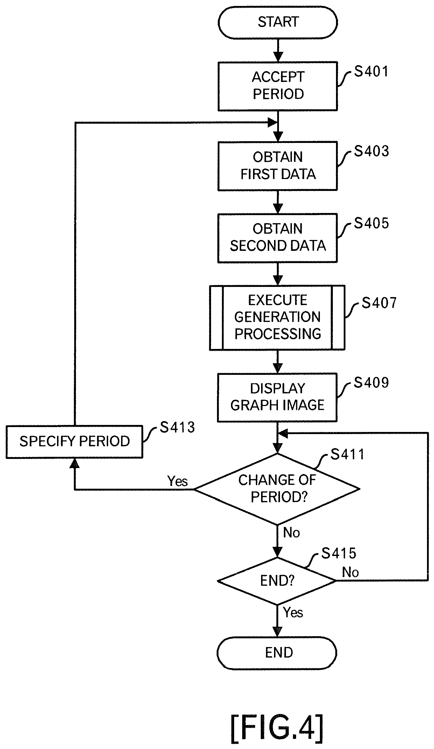

Next, processing in the display processing apparatus 101 will be explained. FIG. 4 illustrates an example of a main processing flow related to the first embodiment. The acceptance unit 301 accepts a period for graphing (S401) . The obtaining unit 303 obtains the first data from the management apparatus 103 (S403). The obtaining unit 303 may obtain the first data from the measurement apparatus 105a or the measurement apparatus 105b. In the case where the display processing apparatus 101 itself generates the first data, the processing of S403 may be omitted. In addition, the obtaining unit 303 may not obtain again the first data that has already been held.

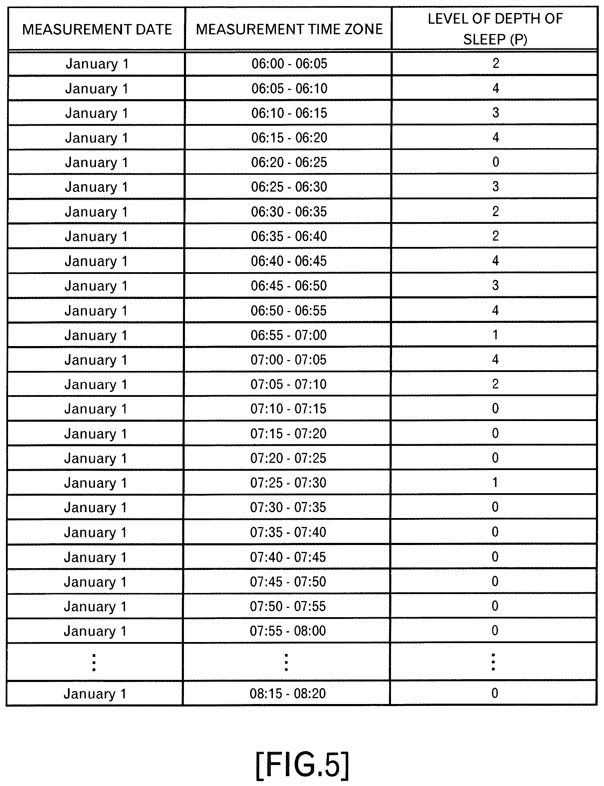

FIG. 5 illustrates an example of the first data. The first data in this example is in a table format. The first data has records for each measurement time zone. A record has a field for storing a measurement date, a field for storing a measurement time zone, and a field for storing the level of the depth of sleep. In this example, the level of the depth of sleep is one of 0 to 4. When the level of the depth of sleep is 0, it represents that the subject is in a non-sleep state. When the level of the depth of sleep is 1 or more, this represents that the subject is in a sleep state. The highest level may be other than 4. The level of the depth of sleep may be represented by a value other than that illustrated in FIG. 5 as long as it represents a difference in the degree of depth.

Returning to the explanation of FIG. 4. The obtaining unit 303 obtains the second data (S405). The obtaining unit 303 may obtain the second data from the measurement apparatus 105a or the measurement apparatus 105b. In the case where the display processing apparatus 101 itself generates the second data, the processing of S405 may be omitted. In addition, the obtaining unit 303 may not obtain again the second data that has already been held.

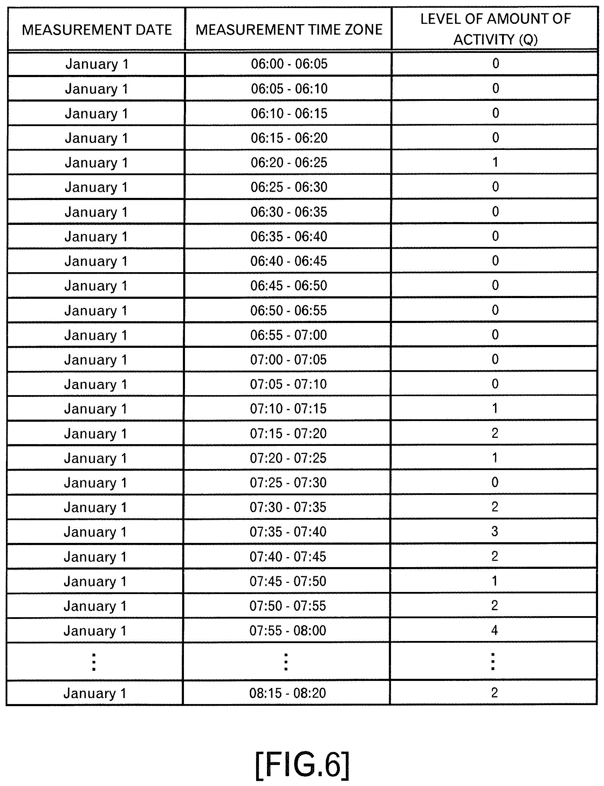

FIG. 6 illustrates an example of the second data. The second data in this example is in a table format. The second data has records for each measurement time zone. A record has a field for storing a measurement date, a field for storing a measurement time zone, and a field for storing the level of the amount of activity. In this example, the level of the amount of activity is one of 0 to 4. When the level of the amount of activity is 1 or more, it represents that the subject is in an active state, in other words, a non-sleep state. For example, when the level of the amount of activity is 1, it represents a state of resting among the non-sleep state. The highest level may be other than 4. When the level of the amount of activity is 0, it represents that the subject is not in an awake state. The level of the amount of activity may be substituted with a value other than the values illustrated in FIG. 6 as long as it is an expression that enables to recognize a difference between a degree of a large amount and a degree of a small amount.

The generator 305 executes generation processing (S407). In the generation processing, the generator 305 generates a graph image. The generated graph image is stored in the frame buffer 321 as image data.

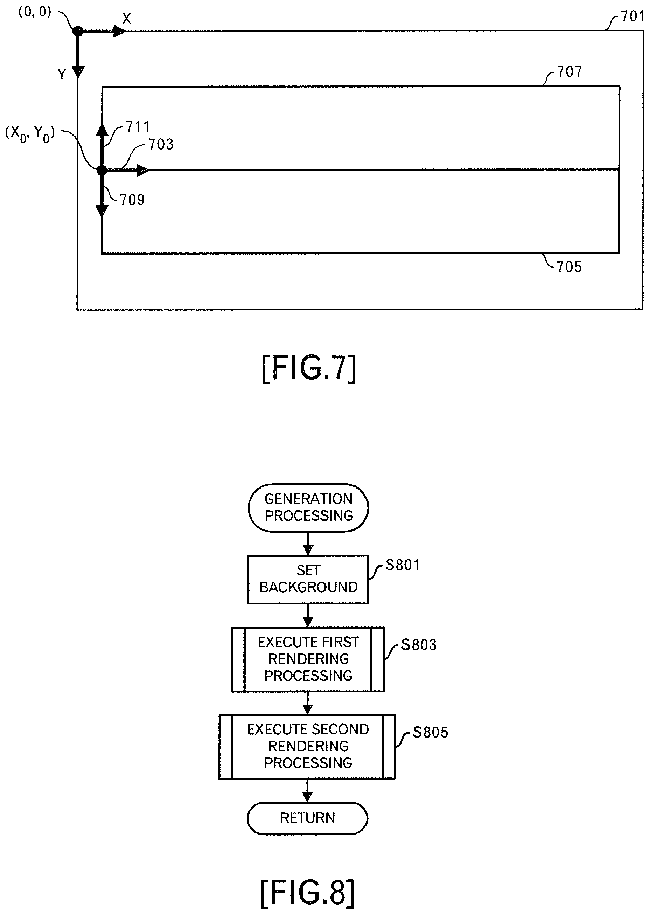

FIG. 7 illustrates an example of configuration of a graph image. The graph image is rendered in the frame 701. The coordinates (X, Y) in the frame 701 are coordinates in a coordinate system with the upper left as its origin. For the X axis, the right direction is positive. For the Y axis, the downward direction is positive.

The origin of the graph is set to the coordinates (X₀, Y₀). An arrow 703 points the positive direction of the time axis 203 in the graph. As illustrated in the figure, the time axis 203 in the graph is provided horizontally.

A bar graph representing the level of the depth of sleep is rendered in a first area 705. A bar graph representing the level of the amount of activity is rendered in a second area 707. The first area 705 and the second area 707 are in contact with each other with the time axis 203 in the graph as a boundary.

One end of the bar 205 representing the level of the depth of sleep is in contact with the time axis 203. As illustrated in FIG. 2, the bar graph representing the level of the depth of sleep represents the level of the depth of sleep by a distance from the time axis 203 to the other end. Therefore, the bar 205 representing the level of the depth of sleep has a range up to a position corresponding to the level of the depth of sleep with the downward direction being positive as pointed by the arrow 709.

One end of the bar 207 representing the level of the amount of activity is also in contact with the time axis 203. As illustrated in FIG. 2, the bar graph representing the level of the amount of activity represents the level of the amount of activity by a distance from the time axis 203 to the other end. Therefore, the bar 207 representing the level of the amount of activity has a range up to a position corresponding to the level of the amount of activity with the upward direction being positive as represented by the arrow 711.

In this way, the graph as a whole represents that the activity is more active as going to one side (in this example, upper side) of the time axis 203, and that the activity is more inactive as going to the other side (lower side in this example) of the time axis 203. And it becomes possible to display an active state and a sleep state as continuous state change in a single graph without breaks. For example, when taking a nap in a time zone in which a state should be the active state, the degree of the depth of sleep and the degree of the amount of activity before and after the nap can be depicted as continuous graphs. Therefore, this is helpful to observe from a viewpoint of an activity condition in which the subject took the nap, a viewpoint of depth of sleep caused by the nap, or a viewpoint of how the subsequent activity condition changed due to the nap.

Next, the generation processing will be explained. FIG. 8 illustrates an example of a generation processing flow. The generator 305 first sets a background image in the frame 701 (S801). At this time, the generator 305 obtains an image data of the background, which is stored in the background storage unit 317, and stores it in the frame buffer 321.

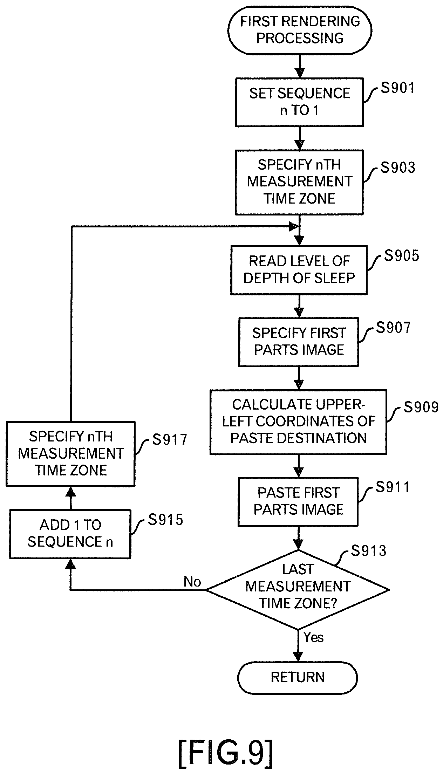

Then, the generator 305 executes the first rendering processing (S803). FIG. 9 illustrates an example of a first rendering processing flow. The generator 305 sequentially processes each measurement time zone included in the period for graphing in chronological order. First, the generator 305 sets a parameter n representing a sequence of a measurement time zone to be processed as 1 (S901). In the following, the measurement time zone to be processed is specif iedby the parameternrepresenting the sequence. The parameter n representing the sequence is stored in the internal data storage unit 311. When the parameter n representing the sequence is 1, it means that the measurement time zone to be processed is the measurement time zone at the head of the period. Therefore, the generator 305 specifies the nth (in other words, first) measurement time zone in the period accepted in S401 (S903).

Then, the generator 305 reads a value P of the level of the depth of sleep, which corresponds to the specified measurement time zone, from the first data stored in the first data storage unit 313 (S905).

The generator 305 specifies the first parts image corresponding to that level of the depth of sleep among parts images stored in the parts storage unit 319 (S907).

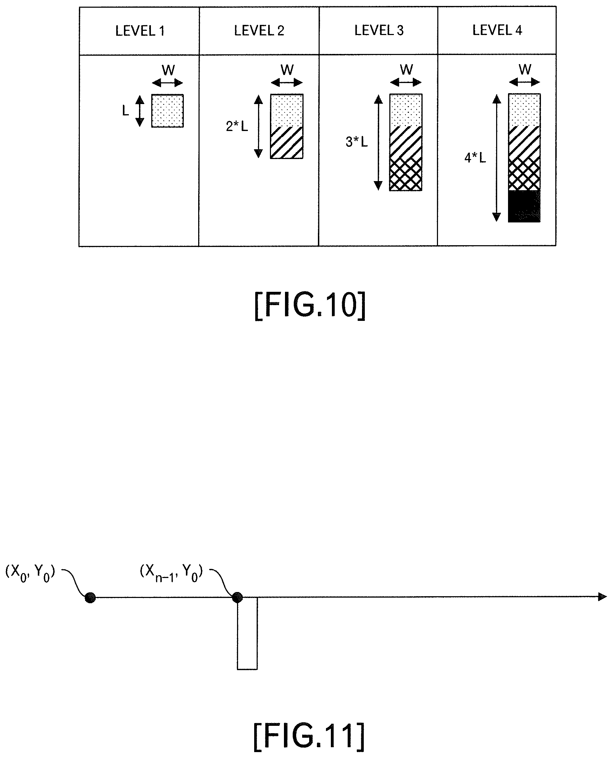

FIG. 10 illustrates an example of the first parts image. The parts storage unit 319 stores a first parts image for each level of the depth of sleep. The first parts image is pasted when rendering a bar 205 representing the level of the depth of sleep. The origin in the coordinate system of the first parts image is, for example, the upper-left end point. A width of the bar 205 representing the level of the depth of sleep is proportional to the measurement time zone, and a length of the bar 205 representing the level of the depth of sleep is proportional to the value of the level of the depth of sleep. In this example, the width of the first parts image which is a rectangle is the predetermined value W, and the length of the first parts image is the value obtained by multiplying the value P of the level of the depth of sleep by a predetermined value L.

Furthermore, the first parts image is divided into areas. And the number of the areas corresponds to the value of the level of the depth of sleep. Each area is divided in the vertical direction. The color occupying each area is set to be darker in stages from the top to the bottom. Therefore, a chroma value of the color occupying the uppermost area is the smallest, the chroma value gradually increases toward the lower area, and the chroma value of the color occupying the lowermost area is the largest. In FIG. 10, an area of a dot pattern has the smallest chroma value, an area of a diagonal line pattern has larger chroma value, an area of a grid pattern has still larger chroma value, and an area of a filled pattern has the largest chroma value. The color hue ("blue" in this example) occupying each area is common.

Furthermore, the first parts image may not be divided into a plurality of areas, and a color with the same strength may occupy the entire surface. In that case, a common color is used for the first parts image corresponding to each level of the depth of sleep.

Returning to the explanation of FIG. 9, the generator 305 calculates the upper-left coordinates of the paste destination (S909). The paste destination of the first parts image will be explained with reference to FIG. 11. An area that corresponds to the nth measurement time zone and on which the first parts image is pasted is represented by a rectangle. The upper-left coordinates of the paste area are (Xₙ₋₁, Y₀), as illustrated. Xₙ₋₁ is found by calculating (n - 1) * W. Y₀ is a predetermined value.

Returning to the explanation of FIG. 9, the generator 305 pastes the first parts image by aligning the origin of the first parts image with the upper-left coordinates calculated in S909 (S911). The data of the first parts image pasted at this time is stored in the frame buffer 321.

Then, the generator 305 determines whether ornot the processed measurement time zone corresponds to the end of the period (S913). When it is determined that the processed measurement time zone does not correspond to the end of the period, the generator 305 adds 1 to the parameter n representing the sequence (S915). The generator 3 05 specifies the nthmeasurement time zone (S917). Then, the processing returns to S905 to repeat the aforementioned processing.

When it is determined that the processed measurement time zone corresponds to the end of the period, the first rendering processing ends.

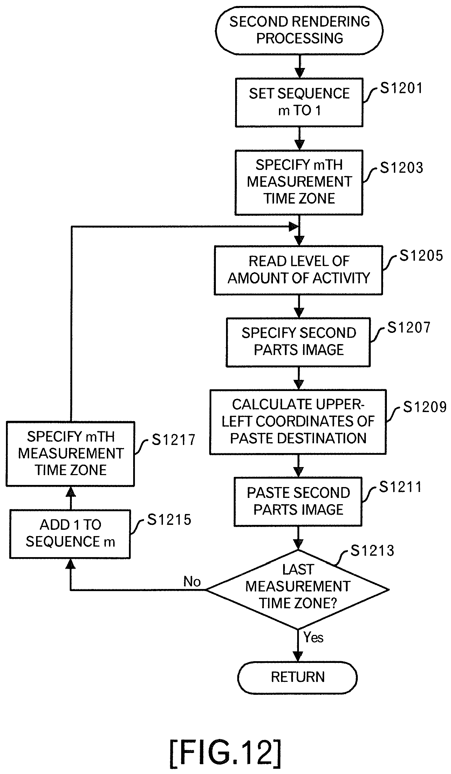

Returning to the explanation of FIG. 8, the generator 305 executes second rendering processing (S805) . FIG. 12 illustrates an example of a second rendering processing flow. The generator 305 sequentially processes each measurement time zone included in the period for graphing in chronological order. First, the generator 305 sets a parameter m representing a sequence of a measurement time zone to be processed to 1 (S1201). In the following description, the measurement time zone to be processed is specified by the parameter m representing the sequence. The parameter m representing the sequence is stored in the internal data storage unit 311. If the parameter m representing the sequence is 1, it means that the measurement time zone to be processed is the measurement time zone at the head of the period. Accordingly, the generator 305 specifies the mth (in other words, first) measurement time zone in the period received in S401 (S1203).

Then, the generator 305 reads a value Q of the level of the amount of activity, which corresponds to the specified measurement time zone, from the second data stored in the second data storage unit 315 (S1205).

The generator 305 specifies a second parts image, which corresponds to the level of the amount of activity, among the parts images stored in the parts storage unit 319 (S1207).

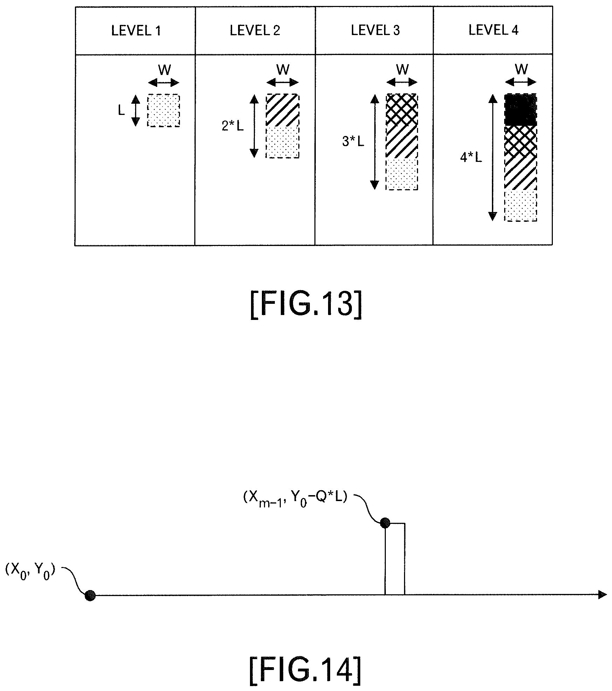

FIG. 13 illustrates an example of the second parts image. The second parts image is pasted when rendering a bar 207 representing the level of the amount of activity. The origin in the coordinate system of the second parts image is, for example, the upper-left end point. The width of the bar 207 representing the level of the amount of activity is proportional to the measurement time zone. And the length of the bar 207 representing the level of the amount of activity is proportional to a value of the level of the amount of activity. In this example, the width of the secondparts image which is a rectangle is a predetermined value W, and the length of the second parts image is the value obtained by multiplying the value Q of the level of the amount of activity by a predetermined value L.

Furthermore, the second parts image is divided into areas. And the number of the areas corresponds to the value of the level of the amount of activity. Each area is divided in the vertical direction. The color occupying each area is set to be darker in stages from the bottom to the top. Therefore, a chroma value of the color occupying the lowermost area is the smallest, the chroma value gradually increases toward the upper area, and the chroma value of the color occupying the uppermost area is the largest. In FIG. 13, an area of a dot pattern has the smallest chroma value, an area of a diagonal line pattern has larger chroma value, an area of a grid pattern has still larger chroma value, and an area of a filled pattern has the largest chroma value. The color hue ("orange" in this example) occupying each area is common.

Moreover, the second parts image may not be divided into a plurality of areas, and a color with the same strength may occupy the entire surface. In that case, a common color is used for the second parts image corresponding to each level of the amount of activity.

Returning to the explanation of FIG. 12, the generator 305 calculates the upper-left coordinates of a paste destination (S1209). The paste destination of the second parts image will be explained with reference to FIG. 14. An area that corresponds to the mth measurement time zone and on which the second parts image is pasted is represented by a rectangle. The upper-left coordinates of the paste area are (Xₘ₋₁, Y₀ - Q*L) as shown. Xₘ₋₁ is found by calculating (m - 1) * W. Y₀ - Q * L is found by subtracting a product of the value of the level of an amount of activity Q and the predetermined value L from the predetermined value Y₀.

Returning to the explanation of FIG. 12, the generator 305 pastes the second parts image by aligning the origin of the second parts image with the upper-left coordinates calculated in S1209 (S1211). Data of the second parts image pasted at this time is stored in the frame buffer 321.

Then, the generator 305 determines whether or not the processed measurement time zone corresponds to the end of the period (S1213). When it is determined that the processed measurement time zone does not correspond to the end of the period, the generator 305 adds 1 to the parameter m representing the sequence (S1215). The generator 305 specifies the mth measurement time zone (S1217). Then, the processing returns to S1205 to repeat the aforementioned processing.

When it is determined that the processed measurement time zone corresponds to the end of the period, the second rendering processing ends and the generation processing illustrated in FIG. 8 also ends. Then, the processing proceeds to S409 illustrated in FIG. 4.

Returning to the description of FIG. 4. The display processing unit 307 performs processing for displaying a graph image (S409). Specifically, the display processing unit 307 causes the display controller 309 controlling the display device to read the image data stored in the frame buffer 321. The display controller 309 lights each pixel of the display device with the color designated in the image data.

Thereafter, the acceptance unit 301 determines whether or not the acceptance unit 301 has accepted an instruction to change the period (S411). When it is determined that the acceptance unit 301 has accepted the instruction to change the period, the acceptance unit 301 specifies a period to be displayed next (S413). For example, when accepting an instruction to display the continuation of the graph, the acceptance unit 301 specifies the subsequent period.

Then, returning to the processing of S403, the display processing apparatus 101 repeats the aforementioned processing for the period specified in S413. In other words, in S403, the obtaining unit 303 obtains the first data corresponding to the period specified in S413. In S405, the obtaining unit 303 obtains the second data corresponding to the period specified in S413. In S407, the generator 305 generates a graph image for the period specified in S413. In step S409, the display processing unit 307 displays the graph image of the period specified in step S413.

On the other hand, when it is determined that the acceptance unit 301 has not accepted an instruction to change the period, the acceptance unit 301 determines whether or not the acceptance unit 301 has accepted an instruction to terminate the processing (S415). When it is determined that the acceptance unit 301 has not accepted the instruction to end the processing, the processing returns to S411. When it is determined that the acceptance unit 301 has accepted the instruction to end the processing, the processing ends.

According to this embodiment, a user can chronologically grasp the relation between the depth of sleep and the amount of activity.

Furthermore, the user can easily and sensuously image the degree of depth of sleep and the degree of the amount of activity. If the degree of the depthof sleep is depicted in the downwarddirection, for example, an impression that consciousness diminishes due to the sleep is likely to be imaged. In addition, if the degree of the amount of activity is depicted in the upward direction, for example, an impression that the consciousness becomes clear by the activity is likely to be imaged.

In addition, if the deep sleep state is depicted in a dark color, the user can easily grasp the depth of sleep in contrast to the non-sleep state or the light sleep state. In addition, if the state with a large amount of activity is depicted in a dark color, the user can easily grasp the intensity of the activity in contrast to the sleep state and the state with a small amount of activity.

### [Embodiment 2]

In the first embodiment, an example in which the entire graph image is rendered in the frame buffer 321 and then the entire graph image is displayed has been explained. However, in the second embodiment, an example in which each parts image is superimposed and displayed will be explained.

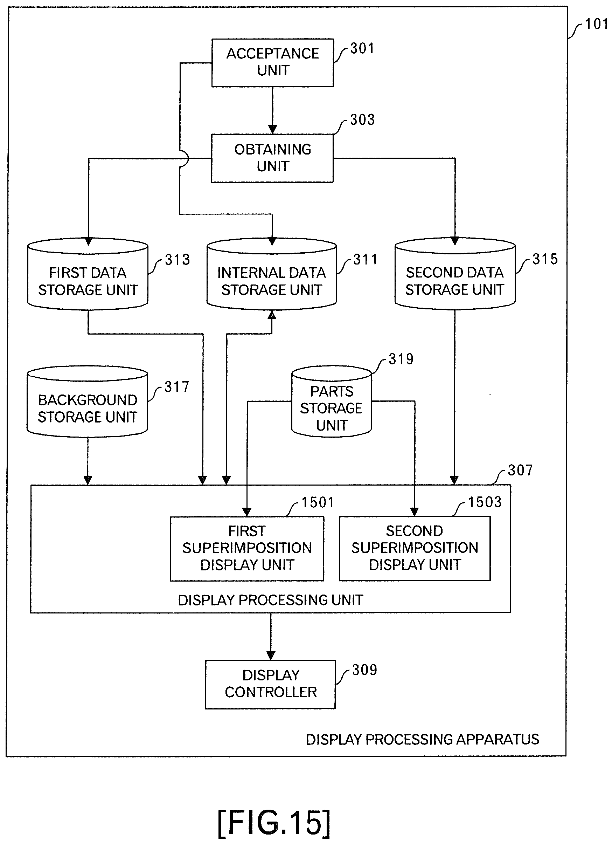

FIG. 15 illustrates an example of module configuration of the display processing apparatus 101 related to the second embodiment. The acceptance unit 301, the obtaining unit 303, the internal data storage unit 311, the first data storage unit 313, the second data storage unit 315, the background storage unit 317 and the parts storage unit 319 are as explained in the first embodiment (FIG. 3) . The display controller 309 related to the second embodiment has a function for superimposing and displaying partial images on a display device in accordance with designated coordinates. Hereinafter, superimposing anddisplayingpartial images on a display device in this way is referred to as a super imposition display. The display processing unit 307 related to the second embodiment performs processing for superimposing and displaying the first parts image and the second parts image by using the function of the superimposition display in the display controller 309. The display processing unit 307 according to the second embodiment has a first superimposition display unit 1501 and a second superimposition display unit 1503. The first superimposition display unit 1501 performs processing for superimposing and displaying the first parts image. The second superimposition display unit 1503 performs processing for superimposing and displaying the second parts image.

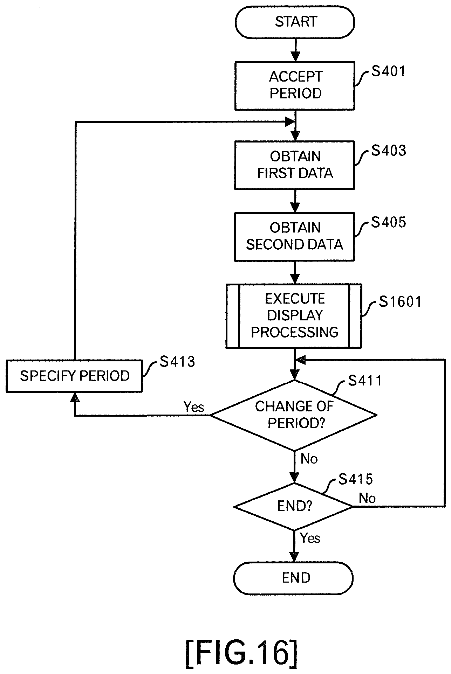

FIG. 16 illustrates an example of a main processing flow related to the second embodiment. The processing in S401 to S405 is as explained in the first embodiment (FIG. 4) . The display processing unit 3 07 executes displayprocessing (S1601). In the second embodiment, the display processing unit 307 performs display processing (A).

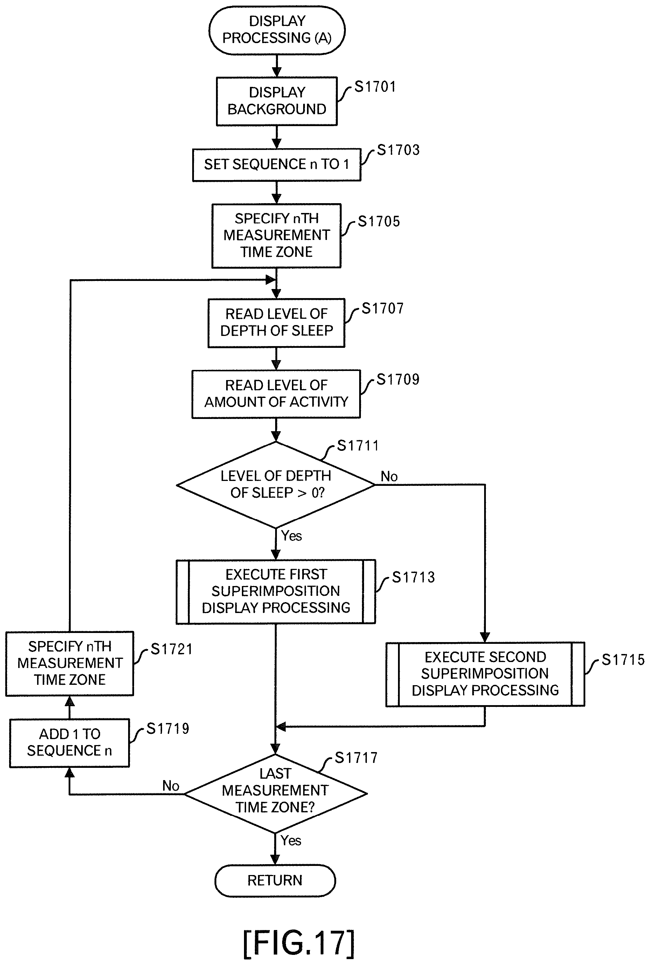

FIG. 17 illustrates an example of the flow of display processing (A) . The display processing unit 307 performs processing for displaying the background image (S1701). At this time, the display processing unit 307 obtains the image data of the background stored in the background storage unit 317, passes the obtained background image data to the display controller 309, and causes the display controller 309 to display it on the display device.

The display processing unit 307 sequentially processes each measurement time zone included in the period for graphing in chronological order. First, the display processing unit 307 sets the parameter n representing a sequence of the measurement time zones to be processed to 1 (S1703). In the following, the measurement time zone to be processed is specified by the parameter n representing the sequence. The parameter n representing the sequence is stored in the internal data storage unit 311. When the parameter n representing the sequence is 1, it means that the measurement time zone tobeprocessed is the measurement time zone at the head of the period. Accordingly, the display processing unit 307 specifies the nth (in other words, the first) measurement time zone in the period received in S401 (S1705).

Next, the display processing unit 307 reads the level of the depth of sleep, which corresponds to the specified measurement time zone, from the first data stored in the first data storage unit 313 (S1707). Furthermore, the display processing unit 307 reads the level of the amount of activity, which corresponds to the specified measurement time zone, from the second data stored in the second data storage unit 315 (S1709).

Then, the display processing unit 307 determines whether or not the level of the depth of sleep is greater than zero (S1711). The level of the depth of sleep, which is greater than 0, represents that the subject is in a sleep state. On the other hand, the level of the depth of sleep, which is 0, represents that the subject is not in a sleep state, that is, a non-sleep state.

When it is determined that the level of the depth of sleep is greater than 0, the display processing unit 307 executes first superimposition display processing (S1713). On the other hand, when it is determined that the level of the depth of sleep is not greater than 0, that is, the level of the depth of sleep is 0, the display processing unit 307 executes second superimposition display processing (S1715).

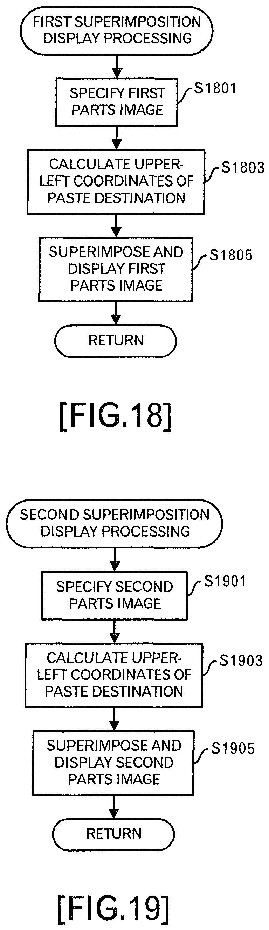

In the first superimposition display processing, the first parts image is superimposed and displayed. FIG. 18 shows an example of a first superimposition display processing flow. The first superimposition display unit 1501 specifies a first parts image corresponding to the level of the depth of sleep, which was read in S1707 among the parts images stored in the parts storage unit 319 (S1801). The first superimposition display unit 1501 calculates the upper-left coordinates of the paste destination in the same way as in the first embodiment (S1803). The first superimposition display unit 1501 designates the upper-left coordinates and causes the display controller 309 controlling the display device to superimpose and display the first parts image (S1805).

In the second superimposition display processing, the second parts image is superimposed and displayed. FIG. 19 illustrates an example of a second superimposition displayprocessing flow. The second superimposition display unit 1503 identifies the second parts image corresponding to the level of the amount of activity read in S1709 among the parts images stored in the parts storage unit 319 (S1901). The second super imposition display unit 1503 calculates the upper-left coordinates of the paste destination as in the first embodiment (S1903). The second super imposition display unit 1503 designates the upper-left coordinates and causes the display controller 309 controlling the display device to superimpose and display the second parts image (S1905).

Returning to the description of FIG. 17. When finishing the first superimposition display processing or the second superimposition display processing, the display processing unit 307 determines whether or not the processed measurement time zone corresponds to the end of the period (S1717). When it is determined that the processed measurement time zone does not correspond to the endof the period, thedisplayprocessingunit 307 adds 1 to the parameter n representing the sequence (S1719). The display processing unit 307 specifies the nth measurement time zone (S1721). Then, the processing returns to S1707 to repeat the aforementioned processing.

When it is determined that the processed measurement time zone corresponds to the end of the period, the display processing (A) ends and the processing shifts to the processing of S411 illustrated in FIG. 16.

The processing of S411 to S415 illustrated in FIG. 16 is as explained in the first embodiment (FIG. 4).

According to this embodiment, a user can chronologically grasp the relation between the depth of sleep and the amount of activity.

In addition, the user can grasp a non-sleep state other than a sleep state.

Furthermore, the user can easily and sensuously image the degree of the depth of sleep and the degree of the amount of activity. If the degree of the depth of sleep is depicted downward, for example, an impression that consciousness diminishes due to the sleep is likely to be imaged. In addition, if the degree of the amount of activity is depicted upward, for example, an impression that the consciousness becomes clear by the activity is likely to be imaged.

In addition, the user can easily and sensuously image the degree of the depth of sleep and the degree of the amount of activity. In addition, if the deep sleep state is depicted in a dark color, the user can easily grasp the depthof sleep in contrast to the non-sleep state or the light sleep state. In addition, if the state with a large amount of activity is depicted in a dark color, the user can easily grasp the intensity of the activity in contrast to the sleep state and the state with a small amount of activity.

### [Embodiment 3]

In the second embodiment, an example of displaying a non-sleep state when it is not in a sleep state is described. However, in the third embodiment, an example of displaying a non-sleep state when it is in an awake state is described.

The module configuration of the display processing apparatus 101 is similar to that in the case of the second embodiment (FIG. 15) . The main processing is also the same as in the case of the second embodiment (FIG. 16).

In the third embodiment, display processing (B) is executed instead of the displayprocessing (A) described in the second embodiment in S1601 shown in FIG. 16.

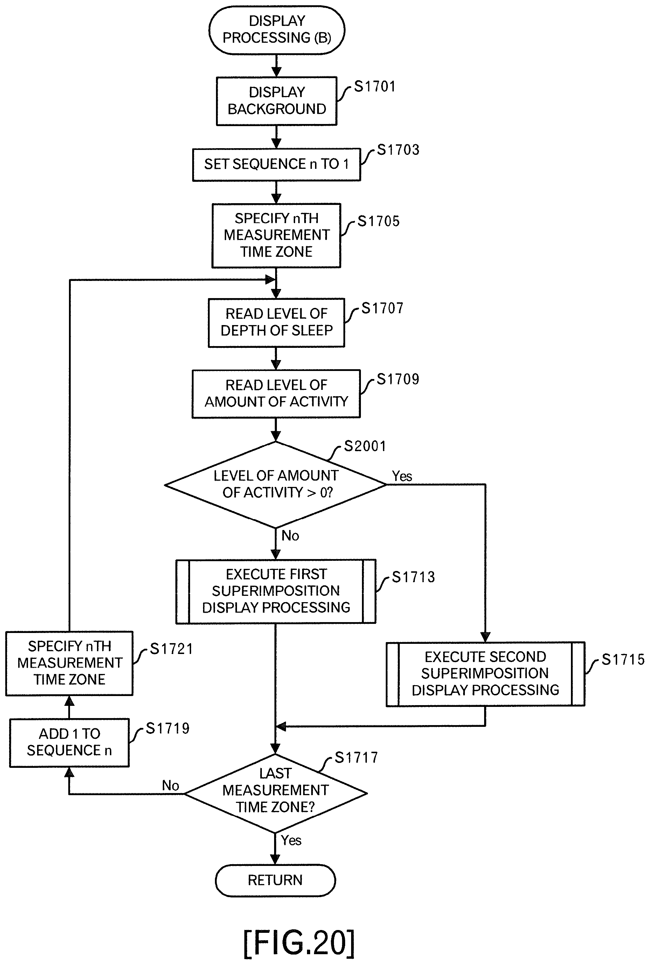

FIG. 20 illustrates an example of a display processing (B) flow. The processing of S1701 to S1709 is as explained in the display processing (A) (FIG. 17).

The display processing unit 307 determines whether or not the level of the activity amount is greater than 0 (S2001). The level of the amount of activity, which is greater than 0, represents that the subject is in an awake state, that is, a non-sleep state.

When it is determined that the level of the amount of activity is not greater than 0, that is, the level of the amount of activity is 0, the displayprocessing unit 3 07 executes the first superimposition display processing (S1713). On the other hand, when it is determined that the level of the amount of activity is larger than 0, the display processing unit 307 executes the second superimposition display processing (S1715).

The first superimposition display processing is also as explained in the second embodiment (FIG. 18). Moreover, the second superimposition display processing is also as explained in the second embodiment (FIG. 19).

Furthermore, the processing of S1717 to S1721 is also as explained in the display processing (A) (FIG. 17).

According to this embodiment, the user can grasp a non-sleep state that is an awake state.

Although the embodiments of this invention were explained above, this invention is not limited to those. For example, the aforementioned functional block configuration does not always correspond to actual program module configuration.

Moreover, the aforementioned configuration of each storage area is a mere example, and may be changed. Furthermore, as for the processing flow, as long as the processing results do not change, the turns of the steps may be exchanged or the steps may be executed in parallel.

In addition, the aforementioned display processing apparatus 101 is a computer apparatus as illustrated in FIG. 21. That is, a memory 2501, a CPU (central processing unit) 2503, a HDD (hard disk drive) 2505, a display controller 2507 connected to a display device 2509, a drive device 2513 for a removable disk 2511, an input unit 2515, and a communication controller 2517 for connection with a network are connected through a bus 2519 as illustrated in FIG. 21. An OS (operating system) and an application program for carrying out the foregoing processing in the embodiments, are stored in the HDD 2505, and when executed by the CPU 2503, they are read out from the HDD 2505 to the memory 2501. As the need arises, the CPU 2503 controls the display controller 2507, the communication controller 2517, and the drive device 2513, and causes them to perform predetermined operations. Moreover, intermediate processing data is stored in the memory 2501, and if necessary, it is stored in the HDD 2505. In these embodiments of this invention, the application program to realize the aforementioned processing is stored in the computer-readable, non-transitory removable disk 2511 and distributed, and then it is installed into the HDD 2505 from the drive device 2513. It may be installed into the HDD 2505 via the network such as the Internet and the communication controller 2517. In the computer apparatus as stated above, the hardware such as the CPU 2503 and the memory 2501, the OS and the application programs systematically cooperate with each other, so that various functions as described above in details are realized.

Moreover, the aforementioned display processing apparatus 101 may be a mobile terminal device. FIG. 22 illustrates an example of hardware configuration of the mobile terminal device. A mobile terminal device has a CPU (Central Processing Unit) 2201, a storage circuit 2203, a wireless communication antenna 2211, a wireless communication control circuit 2213, a speaker control circuit 2215, a speaker 2217, a microphone control circuit 2219, a microphone 2221, a LCD (Liquid Crystal Display) control circuit 2223, a LCD 2225, a touch pad 2227, a key group 2229, a GPS (Global Positioning System) device 2231, a microcontroller 2233, a geomagnetic sensor 2235, an acceleration sensor 2237, a gyro sensor 2239, a barometric pressure sensor 2241, a temperature sensor 2243 and an illuminance sensor 2245.

The CPU 2201 may also include a modem CPU and an application CPU. The storage circuit 2203 has, for example, a ROM (Read Only Memory) 2205, a RAM (Random Access Memory) 2207 and a flash memory 2209. The ROM 2205 stores, for example, a program and preset data for an operating system and the like. The RAM 2207 includes, for example, an area in which a program for an application or the like is expanded. The RAM 2207 also includes an area that temporarily stores data. The flash memory 2209 stores, for example, a program and data to be stored for an application and the like.

The LCD control circuit 2223 operates a clock circuit at a designated operating frequency, and drives an LCD 2225. The LCD 2225 displays a display screen. The touch pad 2227 is, for example, a panel-shaped sensor that is arranged on the display surface of the LCD 2225, and receives instructions by touch operation. More specifically, an integrated LCD 2225 and the touch pad 2227 are used as a touch panel. The hardware keys of the key group 2229 are all provided on part of the housing.

The wireless communication antenna 2211 receives, for example, radio waves according to the cellular communication format, the wireless LAN (Local Area Network) format, the short-range communication format and the like. The wireless communication control unit 2213 performs control of wireless communication according to frequencies used by each communication format. Bycontrollingwireless communication, audio communication for a phone call, or data communication via the Internet is performed.

The speaker control circuit 2215 performs digital/analog conversion related to audio data. The speaker 2217 outputs analog data as sound. The microphone control circuit 2219 performs analog/digital conversion related to audio data. The microphone 2221 converts sound to analog data.

The microcontroller 2233 is connected to the CPU 2201. The geomagnetic sensor 2235, the acceleration sensor 2237, the gyro sensor 2239, the barometric pressure sensor 2241, the temperature sensor 2243, and the illuminance sensor 2245 are connected to the microcontroller 2233. The microcontroller 2233 controls the geomagnetic sensor 2235, the acceleration sensor 2237, the gyro sensor 2239, the barometric pressure sensor 2241, the temperature sensor 2243, and the illuminance sensor 2245. The geomagnetic sensor 2235 measures a magnetic component that includes geomagnetism. The acceleration sensor 2237 measures acceleration. The gyro sensor 2239 detects an attitude of the portable terminal device. The barometric sensor 2241 measures the atmospheric pressure. The temperature sensor 2243 measures the temperature. The illuminance sensor 2245 measures the illuminance.

The aforementioned embodiments are summarized as follows:

A state display method related to one aspect includes: obtaining both first data that represents first temporal change of a degree of a depth of sleep in a sleep state and second data that represents second temporal change of a degree of an amount of activity in a non-sleep state, the first data and the second data being data measured for a subject; generating, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and displaying the generated graph.

In this way, a user can chronologically grasp a relation between a depth of sleep and an amount of activity.

Furthermore, the first side may be a lower part of a screen display with respect to the time axis, and the second side may be an upper part of the screen display with respect to the time axis.

In this way, the user can easily and sensuously image a degree of a sleep depth and a degree of an activity amount. When the degree of the depth of sleep is depicted downward, for example, an impression that consciousness diminishes due to the sleep is likely to be imaged. Moreover, the degree of the amount of activity is depicted upward, for example, an impression that consciousness becomes clear by the activity is likely to be imaged.

Furthermore, a color of the graph may become darker when the graph becomes more away from the time axis toward the first side or the second side.

In this way, the user can easily and sensuously image the degree of depth of sleep and the degree of the amount of activity. When a deep sleep state is depicted in a dark color, the user can easily grasp the depth of sleep in contrast to a non-sleep state or a light sleep state. In addition, if a state with a large amount of activity is depicted in a dark color, the user can easily grasp the intensity of the activity in contrast to the sleep state and/or a state with a small amount of activity.

A state display method related to another aspect includes: displaying a first object that represents a depth of sleep in a first direction perpendicular to a time axis in a case where measurement results of vital activity of a subject represent a sleep state, when displaying temporal transition of states of the subject based on the measurement results; and displaying a second object that represents an intensity of activity in a second direction which is perpendicular to the time axis and is opposite to the first direction in a case where the measurement results represent a non-sleep state.

In this way, a user can chronologically grasp a relation between the depth of sleep and the amount of activity.

The non-sleep state may be a state other than the sleep state or an awake state.

Therefore, the user can grasp the non-sleep state other than the sleep state. Alternatively, the user can grasp the non-sleep state which is the awake state.

Furthermore, the first direction may be a downward direction of a screen display with respect to the time axis, and the second direction may be an upward direction of the screen display with respect to the time axis.

In this way, the user can easily and sensuously image the degree of the depth of sleep and the degree of the amount of activity. When the degree of the depth of sleep is depicted downward, for example, an impression that consciousness diminishes due to the sleep is likely to be imaged. Moreover, when the degree of the amount of activity is depicted upward, for example, the impression that the consciousness becomes clear by the activity is likely to be imaged.

Furthermore, a color of the first object may become darker when the first object becomes more away from the time axis, and a color of the second object may become darker when the second object becomes more away from the time axis.

In this way, the user can easily and sensuously image the degree of the depth of sleep and the degree of the amount of activity. When a deep sleep state is depicted in a dark color, the user can easily grasp the depth of sleep in contrast to the non-sleep state or a light sleep state. In addition, when a state with a large amount of activity is depicted in a dark color, the user can easily grasp the intensity of the activity in contrast to the sleep state and a state with a small amount of activity.

Incidentally, it is possible to create a program causing a processor or a computer to execute the aforementioned processing, and such a program is stored in a computer readable storage medium or storage device such as a flexible disk, CD-ROM, DVD-ROM, magneto-optic disk, a semiconductormemory, andharddisk. Inaddition, the intermediate processing result is temporarily stored in a storage device such as a main memory or the like.

## Claims

1. A state display method executed by a computer, comprising:
obtaining both first data that represents first temporal change of a degree of a depth of sleep in a sleep state and second data that represents second temporal change of a degree of an amount of activity in a non-sleep state, the first data and the second data being data measured for a subject;
generating, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and
displaying the generated graph.

2. The state display method as set forth in claim 1, wherein the first side is a lower part of a screen display with respect to the time axis, and the second side is an upper part of the screen display with respect to the time axis.

3. The state display method as set forth in claim 1, wherein a color of the graph becomes darker when the graph becomes more away from the time axis toward the first side or the second side.

4. A state display method executed by a computer, comprising:
displaying a first object that represents a depth of sleep in a first direction perpendicular to a time axis in a case where measurement results of vital activity of a subject represent a sleep state, when displaying temporal transition of states of the subject based on the measurement results; and
displaying a second object that represents an intensity of activity in a second direction which is perpendicular to the time axis and is opposite to the first direction in a case where the measurement results represent a non-sleep state.

5. The state displaymethodas set forth in claim4, wherein the non-sleep state is a state other than the sleep state or an awake state.

6. The state display method as set forth in claim 4, wherein the first direction is a downward direction of a screen display with respect to the time axis, and the second direction is an upward direction of the screen display with respect to the time axis.

7. The state display method as set forth in claim 4, wherein a color of the first object becomes darker when the first object becomes more away from the time axis, and a color of the second object becomes darker when the second object becomes more away from the time axis.

8. A program that causes a computer to execute a process, the process comprising;
obtaining both first data that represents first temporal change of a degree of a depth of sleep in a sleep state and second data that represents second temporal change of a degree of an amount of activity in a non-sleep state, the first data and the second data being data measured for a subject;
generating, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and
displaying the generated graph.

9. A program that causes a computer to execute a process, the process comprising;
obtaining both first data that represents first temporal change of a degree of a depth of sleep in a sleep state and second data that represents second temporal change of a degree of an amount of activity in a non-sleep state, the first data and the second data being data measured for a subject;
generating, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and
displaying the generated graph.

10. A state display apparatus, comprising;
an obtaining unit that obtains both first data that represents first temporal change of a degree of a depth of sleep in a sleep state and second data that represents second temporal change of a degree of an amount of activity in a non-sleep state, the first data and the second data being data measured for a subject;
a generator that generates, according to time information represented in the first data and the second data, a graph in which the first temporal change represented in the first data is rendered at a first side of a time axis so that greater depth of sleep makes the graph more away from the time axis, and the second temporal change represented in the second data is rendered at a second side of the time axis so that a greater amount of activity makes the graph more away from the time axis; and
a display unit that displays the generated graph.

11. A state display apparatus, comprising;
a first display unit that displays a first object that represents a depth of sleep in a first direction perpendicular to a time axis in a case where measurement results of vital activity of a subject represent a sleep state, when displaying temporal transition of states of the subject based on the measurement results; and
a second display unit that displays a second object that represents an intensity of activity in a second direction which is perpendicular to the time axis and is opposite to the first direction in a case where the measurement results represent a non-sleep state.
